# EUROPEAN PATENT SPECIFICATION

(11) **EP 3 983 797 B1**
(45) Date of publication and mention of the grant of the patent: **24.01.2024**
(21) Application number: 20758362.6
(22) Date of filing: 09.06.2020
(51) Int. Cl.: G01N 33/04, A01J 5/007

(54) **CONTROL FOR A MILK ANALYSIS APPARATUS**
STEUERUNGSEINHEIT FÜR EINE MILCHANALYSEVORRICHTUNG
UNITÉ DE COMMANDE POUR UN APPAREIL D'ANALYSE DE LAIT

(30) Priority: 14.06.2019 SE 1950716
(43) Date of publication of application: 20.04.2022
(73) Proprietor: DeLaval Holding AB, 147 21 Tumba (SE)
(72) Inventor: DALLERUP RASMUSSEN, Claus, 147 21 Tumba (SE); SLAABY, John, 147 21 Tumba (SE)
(74) Representative: DeLaval International AB
(86) International application number: PCT/SE2020/050583
(87) International publication number: WO 2020/251457

(56) References cited:
- WO-A1-2018/236272
- US-A1- 2006 260 939
- US-A1- 2019 082 659

## Description

### TECHNICAL FIELD

This document discloses a control unit for a milk analysis apparatus. More particularly, it is herein presented a control unit of a milk analysis apparatus comprising a first wireless communication device, for communication with a memory device of a cassette via a second wireless communication device comprised in the cassette.

### BACKGROUND

On an animal farm, it is important to keep the animals healthy in order to enhance milk/ meat production, and/ or to monitor when animals are in heat and/ or pregnant, for example. It is important to inseminate animals at an optimal moment in order to successfully fertilise the animal. In case the animal is not successfully inseminated, milk production is affected.

Several biomarker measurements may be made on the animal, such as e.g. measuring levels of progesterone, LDH (Lactate Dehydrogenase), BHB (Beta-Hydroxybutyrat) and urea. Thereby important information concerning e.g. heat detection and/ or pregnancy of the individual animal may be made (based on measured progesterone level), as well as mastitis (based on LDH) and ketosis (based on BHB). Also, the energy balance of the animal may be estimated (based on urea). One example of an apparatus for testing biological samples from animals is disclosed in document US 2006/0260939 A1. The apparatus described therein comprises a detecting unit having a sample inlet and a sample outlet, and a biosensitive sensor comprising a biosensitive medium for indicating the concentration of a biological compound within each biological sample. The biosensitive medium is provided with an active biosensor region. Means expose each region, when in use, to a biological sample thereby detecting the concentration of the biological compound.

Thereby, a farmer/ operator is provided with important information concerning status of each individual animal. However, to perform and analyse biomarker measurements of all individual animals at a farm, e.g. by applying milk samples on prepared dry sticks, and analyse these samples are time consuming for the farmer, who may have to take care of various other important issues. It also put high demands on administrative skills on the farmer to distinguish biomarker measurements from different animals; to keep track on when it is time to repeat the biomarker measurement for each individual animal and when to change biomarker measurement units; maintenance of the biomarker test equipment as well as high demands on cleanliness for not allowing a biomarker measurement of a first animal to be contaminated by biological matters of another animal.

A milk analysis apparatus may be arranged to cooperate with a milk extracting arrangement, for regularly analysing milk samples of the animals, e.g. at or around the moment of a milking session. The milk analysis apparatus may extract a milk sample and provide it on a milk analysis unit such as a dry stick/ lateral flow stick! lateral flow test strip or similar. The milk is typically diluted with a diluent, which also may be used to rinse the tubings between test sessions. The diluent may be provided in a liquid container.

The milk analysis units are maintained in a cassette, for example on a tape in the cassette. Thereby milk analysis units may be easily administrated at the farm by forwarding one milk analysis unit at the time. One milk analysis unit is typically used for each test sample. The cassette may comprise a large amount of milk analysis units, yet the cassette with the milk analysis units has to be exchanged for a new one when all the milk analysis units have been consumed.

It would be desired to find a solution for assisting the farmer in keeping track on usage of the cassettes with the milk analysis units on the farm in different milk analysis apparatus, and to assist him/ her in changing them in due time for avoiding that no milk tests could be made due to lack of milk analysis units. It is also desired not to change the cassette too early before all milk analysis units have been consumed, as it would be an unnecessary waste of resources.

### SUMMARY

It is therefore an object of this invention to solve at least some of the above problems and facilitate for a farmer to measure a biomarker value of a milk sample of an animal.

A biomarker, or biological marker, generally refers to a measurable indicator of some biological state or condition of the animal. The biomarker value measurement may be associated with pregnancy/ reproduction of the animal, health of the animal, and/ or quality of the milk of the animal.

According to a first aspect of the invention, this objective is achieved by a control unit of a milk analysis apparatus, in an agricultural environment. The milk analysis apparatus comprises a first wireless communication device, for communication with a memory device of a cassette via a second wireless communication device comprised in the cassette. The cassette is insertable into the milk analysis apparatus and comprises a predetermined number of milk analysis units for one-time usage. The control unit is configured to determine number of consumed milk analysis units during a milk analysis session. Further, the control unit is configured to transmit information related to the consumed number of milk analysis units during the milk analysis session, to the memory device of the cassette, via the first wireless communication device, for storage in the memory device of the cassette.

The control unit is configured to obtain number of remaining milk analysis units of the cassette from the memory device of the cassette. Also, the control unit is configured to compare the number of remaining milk analysis units with a first trigger level. Furthermore, the control unit is configured to change test modus of the milk analysis apparatus when fewer milk analysis units than the first trigger level remains.

In a first possible implementation of the control unit according to the first aspect, the calculation of the remaining number of milk analysis units comprises obtain number of remaining milk analysis units of the cassette from the memory device of the cassette before performing the test session. The calculation also comprises to estimate remaining number of milk analysis units on the cassette, based on the obtained number of remaining milk analysis units and the number of consumed milk analysis units during the milk analysis session. The transmitted information concerns the estimated remaining number of milk analysis units.

In a second possible implementation of the control unit according to the first aspect, or according to any previously disclosed implementation thereof, the control unit may be configured to compare the number of remaining milk analysis units with a second trigger level, which is smaller than the first trigger level. In addition, the control unit may also be configured to output an alert, encouraging an agricultural manager to replace the cassette when the remaining amount of milk analysis units is lower than or equal to the second trigger level.

In a third possible implementation of the control unit according to the first aspect, or according to any previously disclosed implementation thereof, the milk analysis units are arranged on a tape of the cassette.

In a fourth possible implementation of the control unit according to the first aspect, or according to any previously disclosed implementation thereof, the control unit may be configured to obtain information concerning a dysfunctional milk analysis unit of the cassette from the memory device of the cassette before performing the test session. Also, the control unit may be configured to detect that the subsequent milk analysis units on the tape is dysfunctional. In further addition, the control unit may be configured to forward the tape in position for milk analysis action on the next non- dysfunctional milk analysis units on the tape.

In a fifth possible implementation of the control unit according to the first aspect, or according to any previously disclosed implementation thereof, the control unit may be configured to detect when the cassette is used for the first time. Also, the control unit may be configured to determine a moment in time when the first-time usage of the cassette is detected. The control unit may furthermore be configured to transmit the determined moment in time to the memory device of the cassette, via the first wireless communication device, for storage in the memory device of the cassette.

In a sixth possible implementation of the control unit according to the first aspect, or according to any previously disclosed implementation thereof, the control unit may be configured to determine a current moment in time. Further, the control unit may be configured to check whether the time difference between the stored moment in time of the memory device of the cassette, associated with the first-time usage of the cassette, and the current moment in time exceeds a predetermined time limit. The control unit may in addition be configured to discontinue usage of the cassette when the predetermined time limit is exceeded.

In a seventh possible implementation of the control unit according to the first aspect, or according to any previously disclosed implementation thereof, the control unit may be configured to transmit a blocking sign prohibiting further usage of the cassette, via the first wireless communication device, for storage in the memory device of the cassette, when the predetermined time limit is exceeded.

In an eighth possible implementation of the control unit according to the first aspect, or according to any previously disclosed implementation thereof, the control unit may be configured to output an alert, encouraging an agricultural manager to change the cassette when the predetermined time limit is exceeded by the time period, via an output device.

In a ninth possible implementation of the control unit according to the first aspect, or according to any previously disclosed implementation thereof, the control unit may be configured to activate the first wireless communication device in association with initiating the test session with the milk analysis units of the cassette. The control unit may be configured to deactivate the first wireless communication device when the information concerning the remaining number of milk analysis units has been transmitted.

According to a second aspect of the invention, this objective is achieved by a system in an agricultural environment. The system comprises a control unit according to the first aspect, or any of the possible implementations thereof. Further, the system also comprises a cassette comprising a predetermined number of milk analysis units for one time usage, a memory device and a second wireless communication device, the cassette being insertable into the milk analysis apparatus for information exchange with the control unit via the first wireless communication device, wherein the memory device is configured to receive information concerning remaining number of milk analysis units from the control unit via the second wireless communication device to and store the received information. In addition, the system comprises a milk analysis apparatus comprising a first wireless communication device, for communication with a memory device of the cassette via a second wireless communication device comprised in the cassette. The system comprises a milk extracting arrangement, operating in conjunction with the milk analysis apparatus.

Thereby, by keeping track of the consumption of the milk analysis units on the cassette during a liquid extraction session and repeatedly store this information in the memory device of the cassette, it may be estimated if/ when it is time to change the cassette; and/ or to change test modus; and/ or to automatically order a replacement cassette; also in case the dosing module is inserted into another milk analysis apparatus.

Replacement of the cassette in due time is thereby ascertained. It may also become possible to trace the history of the cassette, i.e. when it was used for the first time, which may facilitate troubleshooting in case of malfunction or reclamation of the milk analysis apparatus. Non-authorised reusage of the cassette is also avoided.

It is hereby avoided that a liquid extraction session is initiated with a cassette which is lacking sufficient number of remaining milk analysis units and/ or which comprises deteriorated milk analysis units. Further, measures may be taken to ascertain that a new cassette is ordered in time and that the cassette is replaced at the right moment to avoid unnecessary waste of resources i.e. cassettes, while not disturbing the planned milk sample testing of the milk analysis apparatus.

Hereby costs, maintenance and work intensity of the farmer associated with management of the milk analysis apparatus is minimised or at least reduced.

Other advantages and additional novel features will become apparent from the subsequent detailed description.

### FIGURES

Embodiments of the invention will now be described in further detail with reference to the accompanying figures, in which:
- **Figure 1**: illustrates an example of an arrangement for measuring a biomarker value of a milk sample of an animal.
- **Figure 2A**: illustrates a cassette inserted into a milk analysis apparatus, according to an embodiment.
- **Figure 2B**: illustrates a section of a tape comprising dry sticks, according to an embodiment.
- **Figure 3A**: illustrates the cassette, a liquid container and the milk analysis apparatus, according to an embodiment.
- **Figure 3B**: illustrates the cassette from a side view.

### DETAILED DESCRIPTION

Embodiments of the invention described herein are defined as a control unit which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

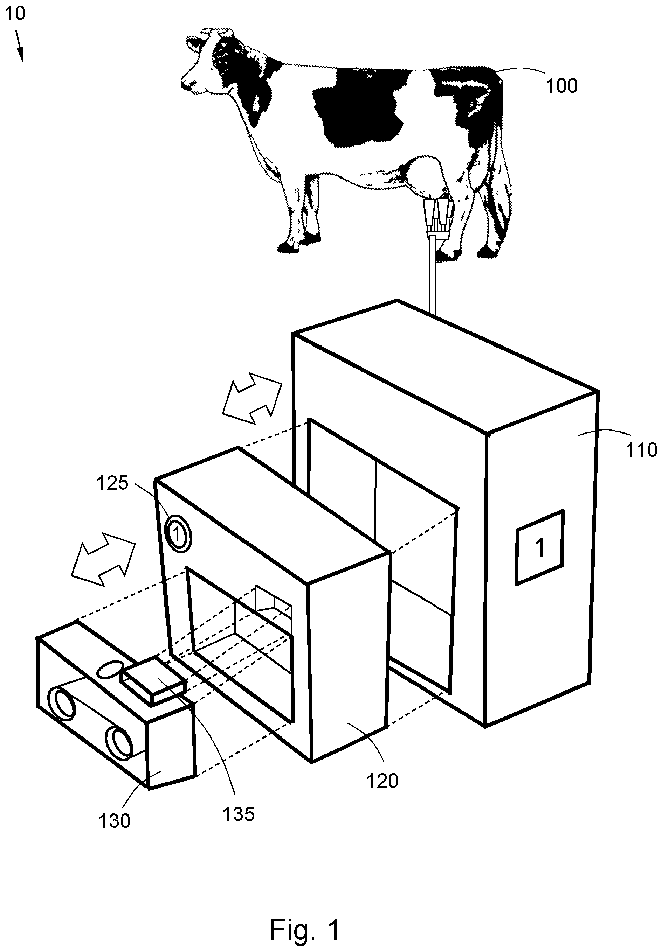

**Figure 1** illustrates a scenario with a system 10 for analysing milk of an animal 100 which may be comprised in a herd of dairy animals at a dairy farm.

"Animal" may be any arbitrary type of domesticated female milk producing and/ or meat producing mammal such as cow, goat, sheep, horse, camel, primate, dairy buffalo, donkey, yak, etc.

Milk of the animal 100 may be extracted by a milk extracting arrangement, or milking equipment 110 such as e.g. a milking robot or other milking arrangement and provided to a milk analysis apparatus 120.

The milk analysis apparatus 120 may be associated with and possibly even releasably insertable into the milk extracting arrangement 110 in some embodiments. Thus, there may be an interface between the milk extracting arrangement 110 and the milk analysis apparatus 120 for providing milk and possibly also electricity via the milk extracting arrangement 110 to the milk analysis apparatus 120.

The milk analysis apparatus 120 comprises various electronics and equipment such as a camera, one or several pumps, a tube element for attachment to the interface to the milk extracting arrangement 110, motors, a communication unit etc.

A cassette 130 is detachably inserted into the milk analysis apparatus 120. The cassette 130 may comprises a tape or similar corresponding arrangement with milk analysis units such as dry sticks/ lateral flow sticks/ lateral flow test strips or similar. The milk analysis units are configured to indicate a biomarker value of a milk sample of the animal 100, e.g. indicate progesterone in the milk sample by a lateral flow test, when a milk sample is applied to the milk analysis unit.

The cassette 130 may in some embodiments be configured to be detachably inserted in the milk analysis apparatus 120 and held in place by a fastening means such as a snap lock, a magnet, a screw, etc., and a door of the milk analysis apparatus 120 may be closed for enclosing the cassette 130 within the milk analysis apparatus 120, thereby further fixating the cassette 130 in the position. It is thereby easy for the farmer to exchange the cassette 130 when all the milk analysis units are consumed, yet the cassette 130 is well protected from the harsh surroundings in an agricultural environment.

Also, a liquid container 135 may be insertable into, or associated with (i.e. physically connected to) the milk analysis apparatus 120. The liquid container 135 may comprise a diluent, which may be used to dilute the extracted milk and also, or alternatively, to rinse the tubing, needle, pump etc., of the milk analysis apparatus 120.

The tubing, needle, pump etc. may be kept in a dosing module. Thereby, the tubing, needle, and pump may be replaced all at the same time in one single replacement.

Thereby, a milk sample of the animal 100 may be extracted from the animal 100 by the milk extracting arrangement 110 and provided via the milk analysis apparatus 120 to one of the milk analysis units on the tape of the cassette 130. The milk analysis units may react on presence and/ or amount of one or several biomarkers, e.g. by changing colours, or intensity of a colour. The camera in the milk analysis apparatus 120 may capture an image through an opening of the cassette 130. The captured image of the milk analysis unit may then be analysed by a control unit, and based on the intensity of the colour, presence and/ or quantity of the biomarker in the milk sample may thereby be determined.

The measured biomarker may be e.g. progesterone, glycoprotein, oestrogen and/ or Gon-adatropin-Releasing Hormones, or any other similar biomarker associated with reproduction or health of the animal 100, in different embodiments.

Progesterone is a hormone that regulates several physiological functions of the animal 100. Progesterone may prepare the uterus for pregnancy, maintain the pregnancy if fertilisation occurs, and inhibit the animal 100 from showing signs of standing oestrus and ovulating when pregnant. Progesterone levels, for example, may rise at the beginning of the pregnancy, and be kept at a high level throughout the pregnancy of the animal 100. Progesterone levels in milk samples may be used to monitor pregnancy, oestrous cycles (heat detection) and/ or postpartum ovarian activity. For these reasons, progesterone levels of animals 100 at the farm is interesting for the farmer to detect and keep track of.

However, the measured biomarker may in some embodiments comprise LDH (Lactate Dehydrogenase), BHB (Beta-HydroxyButyrat), urea, and/ or somatic cell count; or other biomarker related to status of the animal 100. In some embodiments, a plurality of the above enumerated biomarkers may be measured. Alternatively, in some embodiment, the farmer may subscribe to a cassette 130 comprising a certain milk analysis units configured to measure a biomarker, or a set of biomarkers, as selected by the farmer; and/ or different cassettes 130 comprising milk analysis units, e.g. on the tape configured to measure different biomarkers, or sets of biomarkers, during different periods of time of the year.

Thus, the milk analysis apparatus 120 comprises several modules such as the cassette 130, the dosing module and/ or the liquid container 135, which are to be changed for a new respective module at particular time intervals, which may occur at different moments in time for the different modules; or alternatively synchronised with each other.

The cassette 130 with the milk analysis units is to be exchanged when the milk analysis units have been consumed. However, the number of used milk analysis units will be dependent on how often sampling is made, how many milk analysis units that are used for each animal 100 and/ or how many defect milk analysis units there are in the cassette 130. For example, in case the farmer is primarily interested in timing insemination of the animal 100, samples of that animal 100 may be taken only around, or right before, a moment in time when it is predicted that the particular animal 100 is in heat. Animals 100 such as cows and heifers typically go into heat or oestrus every 17 to 24 days (in average 21 days), why the next heat could be roughly predicted based on knowledge of the moment in time of the last heat.

Another farmer may want to take a milk sample on every milking event, e.g. in order to survey health status of a particular animal 100. Also, different cassettes 130 may possibly comprise a different number of milk analysis units. The time period between exchange of the cassettes 130, dosing modules and/ or liquid containers 135 for different milk analysis apparatuses 120 may thereby be different.

The dosing module comprises one or several pumps, such as hose pumps and a tube element for attachment to the milk extracting arrangement 110. The one or several pumps may be configured to act on the tube element for advancing the milk sample from the milk extracting arrangement 110 through the tube element to a needle. The milk sample may then be diluted with diluent from the liquid container 135 in a mixing chamber of the needle (or possibly in a separate mixing chamber and then provided to the needle), where after the diluted milk sample may be applied from the needle to the milk analysis unit of the cassette 130.

The various modules, i.e. the dosing module, the liquid container 135 and/ or the cassette 130 may comprise fastening means, e.g. in form of a snap fit arrangement, magnetics, screw joints, etc., arranged to attach the respective module onto the milk analysis apparatus 120.

The modular structure of the provided solution has several advantages. By keeping the arrangement modular in form of the dosing module, the liquid container 135 and the cassette 130, which may be attached to milk extracting arrangement 110 of the farm; costs, maintenance and work intensity of the farmer may be minimised or at least reduced. Also, by separating the consumable material such as milk analysis units/ measurement sticks of the cassette 130, from elements subject to wear, like the pumps of the dosing module, and the electronics and instruments of the milk analysis apparatus; the cassette 130 could be continuously replaced with another replacement cassette e.g. ordered via a courier service or postal office subscription.

The milk analysis apparatus 120 may on the other hand be detached from the milk extracting arrangement 110 and the dosing module/ liquid container 135/ cassette 130 upon malfunction and sent to a workshop for troubleshooting, repair, maintenance, etc.

Meanwhile, an identical replacement milk analysis apparatus 120 may be provided to the farm, enabling continuous biomarker measurements on the farm, also when the equipment of the milk analysis apparatus 120 is malfunctioning, to which the old dosing module / liquid container 135/ cassette 130 could be applied.

Further, the described arrangement may be operated by the farmer without requiring a particularly trained technician to come and visit the farm. Instead, the farmer may send the malfunctioning module to the workshop; or just replace it.

By maintaining the diluent, the milk analysis units and the tubings, respectively, in separate modules, they are protected from any possible affection of the environment at the farm.

However, an appearing problem is that it may become problematic for the farmer to keep track on which module to change at which moment in time. It would be desired to find a tool for reminding the farmer of when to change the respective modules, or at least one particular of the modules; and/ or to make it impossible to run the milk analysis apparatus 120 when there is no more unused milk analysis units in the cassette 130.

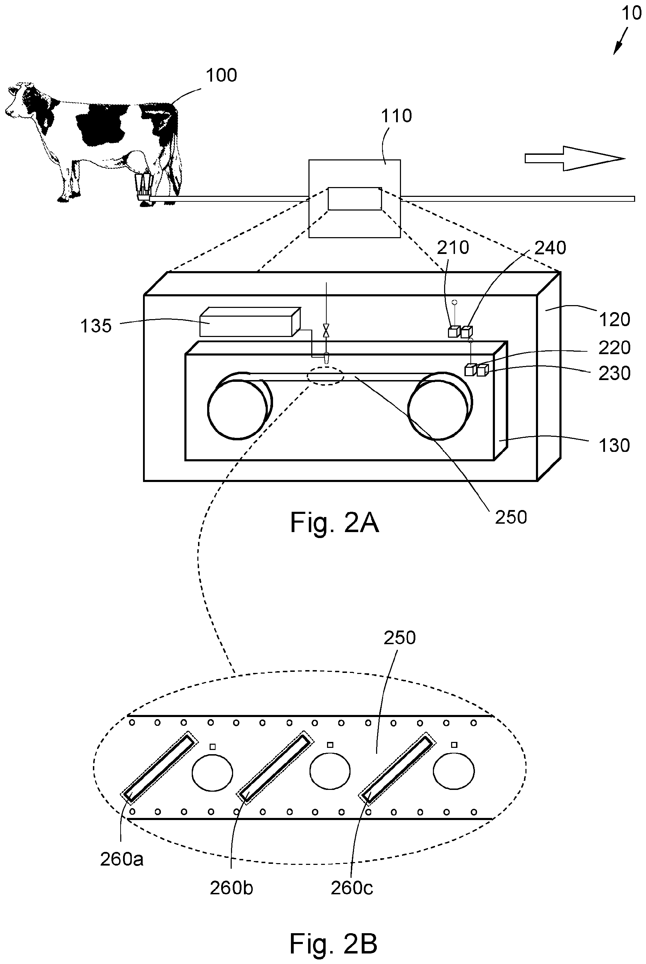

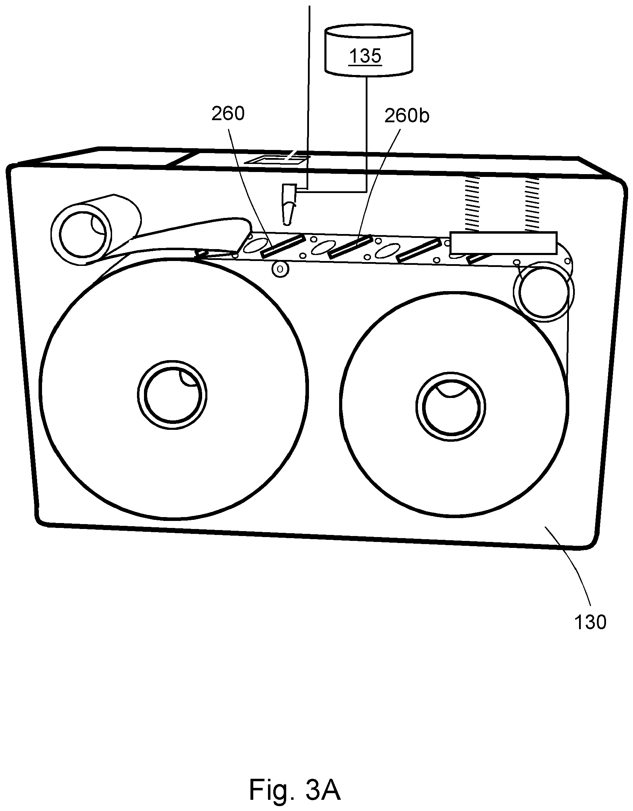

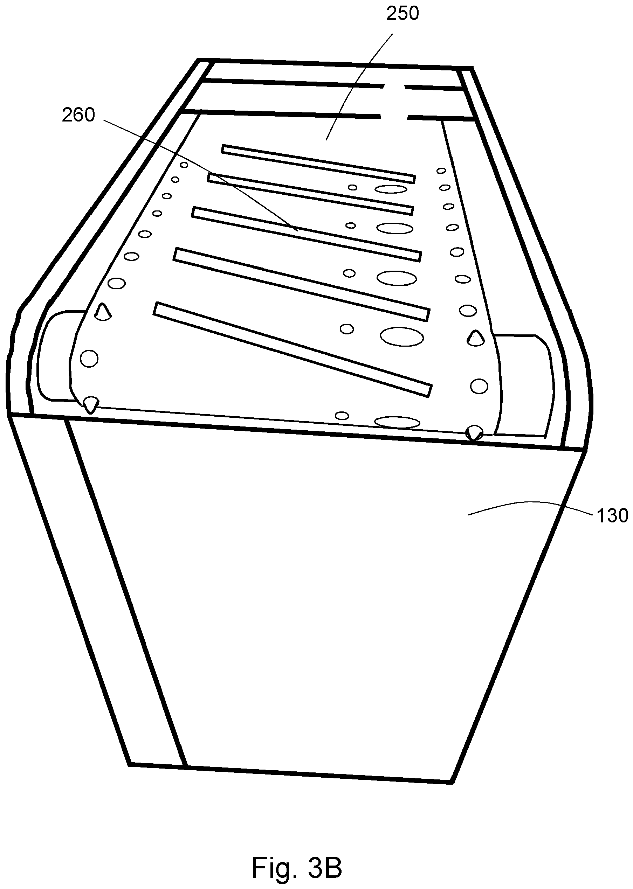

Figure 1 and Figure 2A depict general overviews of the environment in which the milk analysis apparatus 120, the cassette 130, the liquid container 135, and the dosing module according to the provided solution is intended to operate, without going too much into details, in order for the reader to get a rough overview. Sublime examples of details of the tape may be studied in Figure 2B. Figure 3A illustrates a particular aspect of the provided solution and interaction between various modules of the milk analysis apparatus 120, in particular the cassette 130 while Figure 3B illustrates another view of the cassette 130.

**Figure 2A and 2B** illustrates a scenario illustrating a milk analysis apparatus 120, a cassette 130, a liquid container 135 and a dosing module interacting with each other and with the milk extracting arrangement 110, according to an embodiment.

The milk analysis apparatus 120 may comprise electronics and equipment such as e.g. a camera, a tube element for attachment to the milk extracting arrangement 110, a motor, a communication unit, etc., to be used for determining a biometric value of a milk sample received from an animal 100. In some embodiments, one or several pumps and tubings are comprised in the dosing module. The pump/s is configured to act on the tube element for advancing the milk sample from the milk extracting arrangement 110 through the tube element to reach the needle; or the mixing chamber of the needle. The mixing chamber may alternatively be external to the needle. The tube elements are configured to receive the milk sample of the animal 100 via a milk extracting arrangement 110 and provide the milk sample to a needle, i.e. the needle comprised in the dosing module.

In the illustrated embodiment, the dosing module may comprise a needle for applying the milk sample to a milk analysis unit 260a, 260b, 260c on a tape 250 in the cassette 130 through an opening in the cassette 130. The milk analysis units 260a, 260b, 260c may also be referred to as dry sticks, lateral flow sticks, rapid lateral flow test strips, etc. The camera may then align the needle with the milk analysis unit 260a, 260b, 260c on the tape 250 of the cassette 130, in an embodiment. The milk analysis units 260a, 260b, 260c may not necessarily be kept on a tape 250, but other similar solutions may be applied wherein the milk analysis units 260a, 260b, 260c may be maintained on another similar substrate.

The cassette 130 comprises a known number of milk analysis units 260a, 260b, 260c such as e.g. 400, or 500, etc., and/ or a number of milk analysis units 260a, 260b, 260c which is sufficient for supplying testing during a certain time period such as two weeks, a month, etc.

The milk analysis units 260a, 260b, 260c may for example comprise individually sealed lateral flow sticks. The lateral flow sticks may be arranged to indicate a biomarker in the milk sample of the animal 100 when a lateral flow test is performed. The lateral flow sticks may comprise a sample pad with antibody treated gold particles which are dispersed into the milk sample when applied onto the sample pad and an absorbent pad for receiving a capillary flow of the milk sample, from the sample pad. The absorbent pad in turn may comprise a test line treated with a biomarker reference which binds antibody treated gold particles of the milk sample and thereby brings the test line to change colour when exposed for milk comprising a biomarker level lower than a threshold limit. The absorbent pad may also comprise a control line treated with an antibody reference which binds antibody treated gold particles of the milk sample regardless of the progesterone level in the milk, and thereby brings the control line to change colour when exposed for milk comprising antibody treated gold particles.

The dosing module may also comprise a liquid evacuator or drainage, which may collect liquid that has been output by the needle. The liquid, when comprising merely milk, may be returned back to the milk line/ milk extracting arrangement 110 in some embodiments. In other embodiments, when the milk has been mixed with diluent, the liquid may be conveyed away from the cassette 130 in order not to soak or contaminate other, unused, milk analysis units 260a, 260b, 260c of the tape 250 on the cassette 130.

The camera may capture an image of the milk analysis unit 260a, 260b, 260c of the carrier tape 250 through the opening, and based on these images, a cassette external motor may adjust the tape 250 for positioning a new milk analysis unit 260a, 260b, 260c, on which a new test is to be made, in relation to the needle.

The milk analysis apparatus 120 may also comprise a communication device which may communicate via a wired or wireless communication interface with an output unit, a database, a communication device of a farmer, etc.

The control unit may be configured to determine a biomarker value of the milk sample of the animal 100, based on an analysis of the image, captured by the camera. The control unit may be comprised in the milk analysis apparatus 120 in some embodiments; or be external to the milk analysis apparatus 120.

A database may store measured biometric values of the animal 100, associated with an identity reference of the animal 100 and/ or a time stamp of the measurement. Other measurements and/ or data related to the animal 100 may also be stored in the database, such as milk yield, e.g. measured by the milk flow meter, activity, breed, parity, rumination, lactation, resting, feed intake, energy balance, Days In Milk, milk production, age and possibly other similar animal status related parameters.

When a deviation, exceeding a first threshold limit, is detected between the outcomes of the biomarker measurement and the corresponding reference value, an alert may be outputted to the farmer or other responsible person. The alert may comprise e.g. visual information, an audio message, a tactile signal or a combination thereof, encouraging the farmer to further investigate the reasons for the detected deviation in result. In case a plurality of people is working with the herd, a broadcast may be made to the plurality of farmers and their respective associated output units, in some embodiments.

The milk analysis apparatus 120 comprises a first wireless communication device 210. The first wireless communication device 210 is configured for wireless communication with a second wireless communication device 220 comprised in the cassette 130. The wireless communication between the devices 210, 220 may be made by e.g. Near Field Communication (NFC) communication, Bluetooth, Radio-Frequency Identification (RFID) or other similar short-range wireless communication.

The milk analysis apparatus 120 also comprises a control unit 240 while the cassette 130 comprises a memory device 230 for storage of data. The control unit 240 may optionally be situated outside of the milk analysis apparatus 120 yet being in communicational contact with the first wireless communication device 210.

The control unit 240 of the milk analysis apparatus 120 thereby obtains information from the cassette 130, which is stored in the memory device 230 of the cassette 130 and/ or provides information to the memory device 230 of the cassette 130, for storage therein.

**Figure 3A** illustrates the interaction between the milk analysis apparatus 120, the cassette 130, the liquid container 135 and/ or the dosing module.

The cassette 130 is insertable into the milk analysis apparatus 120 and comprises a predetermined number of milk analysis units 260a, 260b, 260c for one-time usage. The milk analysis units 260a, 260b, 260c may for example be arranged on a tape 250 of the cassette 130. The control unit 240 of the milk analysis apparatus 120 is configured to determine a number of consumed milk analysis units 260a, 260b, 260c of the cassette 130 during a milk analysis session. The number of consumed milk analysis units 260a, 260b, 260c may be e.g. 1, 2, 3, ... , etc., depending on the kind of milk analysis that is performed, and/ or depending on number of defect milk analysis units 260a, 260b, 260c that are bypassed.

The control unit 240 is also configured to transmit information related to the consumed number of milk analysis units 260a, 260b, 260c during the milk analysis session, to the memory device 230 of the cassette 130, via the first wireless communication device 210, for storage in the memory device 230 of the cassette 130.

The information related to the consumed number of milk analysis units 260a, 260b, 260c may for example be the number of consumed milk analysis units 260a, 260b, 260c in some embodiments. In other embodiments, the information may comprise a calculated number of remaining milk analysis units 260a, 260b, 260c on the cassette 130.

Hereby, the memory device 230 of the cassette 130 is continuously updated with the concurrent information concerning the number of consumed milk analysis units 260a, 260b, 260c on the cassette 130. By storing this number in the cassette 130, it could at a later moment be provided to the control unit 240 of the milk analysis apparatus 120, also when the cassette 130 has been swapped into another milk analysis apparatus 120. The control unit 240 is then enabled to determine how many milk analysis units 260a, 260b, 260c there are left on the cassette 130 and change test modus of the milk analysis apparatus 120 when fewer milk analysis units 260a, 260b, 260c than a trigger level remains on the cassette 130, such as for example 10-30 milk analysis units 260a, 260b, 260c, and possibly also take further appropriate measures based there upon; for example alerting the farmer when the remaining number of milk analysis units 260a, 260b, 260c is lower than a threshold limit and/or automatically order a replacement cassette 130 when the remaining number of milk analysis units 260a, 260b, 260c is lower than another threshold limit.

The control unit 240 may be configured to calculate the remaining number of milk analysis units 260a, 260b, 260c by: obtain number of remaining milk analysis units 260a, 260b, 260c of the cassette 130 from the memory device 230 of the cassette 130 before performing the test session. Also, the remaining number of milk analysis units 260a, 260b, 260c on the cassette 130 may be estimated, based on the obtained number of remaining milk analysis units 260a, 260b, 260c and the number of consumed milk analysis units 260a, 260b, 260c during the milk analysis session. The transmitted information may then concern the estimated remaining number of milk analysis units 260a, 260b, 260c.

By calculating the remaining number of milk analysis units 260a, 260b, 260c the control unit 240 becomes aware of how many milk analysis sessions that could be performed before changing the cassette 130. In some embodiments, a comparison may be made with a threshold limit and perform different actions depending on the remaining number of milk analysis units 260a, 260b, 260c.

The control unit 240 is configured to obtain number of remaining milk analysis units 260a, 260b, 260c of the cassette 130 from the memory device 230 of the cassette 130 and compare the number of remaining milk analysis units 260a, 260b, 260c with a first trigger level. The control unit 240 is further configured to change test modus of the milk analysis apparatus 120 when fewer milk analysis units 260a, 260b, 260c than the first trigger level remains.

When there is only a limited amount of milk analysis units 260a, 260b, 260c left in the cassette 130, only the most essential test sessions may be performed. Instead of testing all biomarkers at every milk session of every animal 100 visiting the milk analysis apparatus 120, only certain biomarkers (or one single biomarker); and/ or on certain animals 100; and/ or during certain milking sessions, such as e.g. every second milking session, every third milking session, etc. Hereby, the most important testis (as preconfigured or defined by the farmer) could be performed, also in a situation when the milk analysis apparatus 120 is about to run out of milk analysis units 260a, 260b, 260c.

The control unit 240 may be configured to compare the number of remaining milk analysis units 260a, 260b, 260c with a second trigger level, which is smaller than the first trigger level.

The control unit 240 may also be configured to output an alert, encouraging an agricultural manager to replace the cassette 130 when the remaining amount of milk analysis units 260a, 260b, 260c is lower than or equal to the second trigger level. The alert may comprise information enabling the agricultural manager/ farmer to identify the milk analysis apparatus 120 comprising the cassette 130 to be replaced.

The agricultural manager/ farmer is thereby alerted concerning the requirement of replacing the cassette 130, eliminating or at least reducing the risk of running out of milk analysis units 260a, 260b, 260c on a cassette 130 in one of the milk analysis apparatus 120 therein.

In yet some embodiments, the control unit 240 may be configured to automatically order a replacement cassette 130 from a provider, to be delivered to the farm. Thereby, the work of the agricultural manager/ farmer is further facilitated.

The control unit 240 may in addition be configured to obtain information concerning a dysfunctional milk analysis unit 260a, 260b, 260c of the cassette 130 from the memory device 230 of the cassette 130 before performing the test session. The information may for example comprise sequential number of the dysfunctional milk analysis unit 260a, 260b, 260c on the tape 250; an identity reference of the dysfunctional milk analysis unit 260a, 260b, 260c, etc., enabling the control unit 240 to detect it. The control unit 240 may in addition be configured to detect that the subsequent milk analysis units 260a, 260b, 260c on the tape 250 is dysfunctional, e.g. based on the obtained information. Furthermore, the control unit 240 may be configured to forward the tape 250 in position for milk analysis action on the next non- dysfunctional milk analysis units 260a, 260b, 260c on the tape 250, upon detection of the dysfunctional milk analysis unit 260a, 260b, 260c.

When the milk analysis unit 260a, 260b, 260c are produced and arranged on the tape 250, the mounting of the milk analysis unit 260a, 260b, 260c sometimes fail due to various imperfections. When the malfunctioning milk analysis unit 260a, 260b, 260c is mounted on the tape 250 by gluing or taping it thereto, it cannot be removed therefrom without destroying the tape 250, leading to that the whole tape 250 with the already mounted milk analysis units 260a, 260b, 260c has to be wasted. By instead detecting the malfunctioning milk analysis unit 260a, 260b, 260c during the production process and store information thereof in the memory device 230, the tape 250 may be used although one or some milk analysis units 260a, 260b, 260c are dysfunctional, resources are saved and the production process is simplified.

In addition, the control unit 240 may be configured to detect when the cassette 130 is used for the first time. Also, the control unit 240 may be configured to determine a moment in time when the first-time usage of the cassette 130 is detected. The control unit 240 may also be configured to transmit the determined moment in time to the memory device 230 of the cassette 130, via the first wireless communication device 210, for storage in the memory device 230 of the cassette 130.

The control unit 240 may then in some embodiments be configured to determine a current moment in time, e.g. by a time/ calendar functionality, a time providing service or similar means. Also, the check whether the time difference between the stored moment in time of the memory device 230 of the cassette 130, associated with the first-time usage of the cassette 130, and the current moment in time exceeds a predetermined time limit. The predetermined time limit may be set to guarantee that the milk analysis units 260a, 260b, 260c on the cassette 130 are not so old that the functionality is endangered. This may occur for example if the cassette 130 initially is used but then has a time gap of inactivity e.g. due to a malfunctioning milk analysis apparatus 120, or for any other reason. The allowed lifetime of the cassette 130 when inserted in the milk analysis apparatus 120 may be estimated to e.g. 1-2 months.

The control unit 240 may be further configured to discontinue usage of the cassette 130 when the predetermined time limit is exceeded. By storing this determined moment in time of first-time usage in the memory device 230 of the cassette 130 instead of in the milk analysis apparatus 120, the first-time usage of the cassette 130 may be determined in any milk analysis apparatus 120.

The control unit 240 may also be configured to transmit a blocking sign prohibiting further usage of the cassette 130, via the first wireless communication device 210, for storage in the memory device 230 of the cassette 130, when the predetermined time limit is exceeded.

By storing the blocking sign in the memory device 230 of the cassette 130, it is ascertained that the cassette 130 cannot be used when it is too old (since the first-time usage) also when inserted in another milk analysis apparatus 120.

The control unit 240 may be configured to output an alert, encouraging an agricultural manager to change the cassette 130 when the predetermined time limit is exceeded by the time period, via an output device, such as a mobile phone, computing device, augmented reality device, smart watch of the farmer, etc.

By alerting the agricultural manager/ farmer, he/ she becomes aware of the situation with the cassette 130 and is reminded to exchange the cassette 130 of the particular milk analysis apparatus 120, thereby relieving him/ her from the tedious work of continuously monitoring the respective status of the cassettes 130 of different milk analysis apparatus 120 at the farm.

In yet some embodiments, the control unit 240 may be configured to activate the first wireless communication device 210 in association with initiating the test session with the milk analysis units 260a, 260b, 260c of the cassette 130. Also, or alternatively, the control unit 240 may be configured to deactivate the first wireless communication device 210 when the information concerning the remaining number of milk analysis units 260a, 260b, 260c has been transmitted.

It thereby becomes possible to keep the wireless communication devices 210, 220 activated for as brief period of time as possible. Thereby, transmission disturbance on other wireless communication devices close-by is avoided or at least reduced. Also, energy is saved. **Figure 3B** illustrates the cassette 130 with the tape 250 comprising milk analysis units 260a, 260b, 260c on the tape 250.

The cassette 130 comprises a predetermined number of milk analysis units 260a, 260b, 260c for one time usage, a memory device 230 and a second wireless communication device 220, which cassette 130 is insertable into a milk analysis apparatus 120, for information exchange with a control unit 240 of the milk analysis apparatus 120 via a first wireless communication device 210 of the milk analysis apparatus 120, when the cassette 130 is inserted into the milk analysis apparatus 120.

The memory device 230 is configured to receive information concerning remaining number of milk analysis units 260a, 260b, 260c from the control unit 240 of the milk analysis apparatus 120, via the second wireless communication device 220. The memory device 230 is also configured to store the received information.

The memory device 230 of the cassette 130 is configured to provide number of remaining milk analysis units 260a, 260b, 260c of the cassette 130 to the control unit 240, via the second wireless communication device 220.

The memory device 230 may in some embodiments be configured to store information concerning a dysfunctional milk analysis unit 260a, 260b, 260c of the cassette 130. Also, the memory device 230 may be configured to provide the information concerning the dysfunctional milk analysis unit 260a, 260b, 260c to the control unit 240, via the second wireless communication device 220.

The memory device 230 may additionally be configured to receive information concerning a moment in time of a first-time usage of the cassette 130 from the control unit 240 of the milk analysis apparatus 120, via the second wireless communication device 220. The memory device 230 may be further configured to store the received information.

Also, the memory device 230 may be configured to provide stored information concerning the moment in time of the first-time usage of the cassette 130 to the control unit 240, via the second wireless communication device 220.

It is hereby avoided that a cassette 130 which has been inactive for too long time (i.e. a time period exceeding the predetermined time limit) is inserted and used in a milk analysis apparatus 120, as the period of inactivity may have caused deterioration of milk analysis units 260a, 260b, 260c of the cassette 130.

The memory device 230 may furthermore be configured to receive a blocking sign prohibiting further usage of the cassette 130 from the control unit 240 of the milk analysis apparatus 120, via the second wireless communication device 220. The memory device 230 may also be configured to store the received information.

The memory device 230 may in some embodiments be configured to receive a blocking sign prohibiting further usage of the cassette 130, from the control unit 240 via the second wireless communication device 220. Also, the memory device 230 may be configured to store the received blocking sign, thereby prohibiting further usage of the cassette 130.

In some embodiments, the memory device 230 of the cassette 130 may be configured to store production information, such as batch number and/ or date of production. Hereby, later troubleshooting of errors of the milk analysis apparatus 120 may be facilitated. Also, cassettes 130 of an erroneous batch may be detected and sorted out for replacement.

The embodiments, or parts thereof, illustrated in Figure 1, Figure 2A, Figure 2B, Figure 3A, and/ or Figure 3B may with advantage be combined with each other for achieving further benefits.

The terminology used in the description of the embodiments as illustrated in the accompanying drawings is not intended to be limiting of the described control unit 240, the dosing module, liquid container 135, cassette 130, milk analysis apparatus 120 and/ or system 10. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware but may also be distributed in other forms such as via Internet or other wired or wireless communication system.

## Claims

1. A control unit (240) for a milk analysis apparatus (120), wherein the milk analysis apparatus (120) comprises a first wireless communication device (210), for communication with a memory device (230) of a cassette (130) via a second wireless communication device (220) comprised in the cassette (130); wherein the cassette (130) is insertable into the milk analysis apparatus (120) and comprises a predetermined number of milk analysis units (260a, 260b, 260c) for one time usage; **characterized in that** the control unit (240) is configured to:
determine a number of consumed milk analysis units (260a, 260b, 260c) during a milk analysis session;
transmit information related to the consumed number of milk analysis units (260a, 260b, 260c) during the milk analysis session, to the memory device (230) of the cassette (130), via the first wireless communication device (210), for storage in the memory device (230) of the cassette (130);
obtain number of remaining milk analysis units (260a, 260b, 260c) of the cassette (130) from the memory device (230) of the cassette (130);
compare the number of remaining milk analysis units (260a, 260b, 260c) with a first trigger level; and
change test modus of the milk analysis apparatus (120) when fewer milk analysis units (260a, 260b, 260c) than the first trigger level remains.

2. The control unit (240) according to claim 1, wherein a calculation of a remaining number of milk analysis units (260a, 260b, 260c) comprises:
obtain number of remaining milk analysis units (260a, 260b, 260c) of the cassette (130) from the memory device (230) of the cassette (130) before performing the test session;
estimate remaining number of milk analysis units (260a, 260b, 260c) on the cassette (130), based on the obtained number of remaining milk analysis units (260a, 260b, 260c) and the number of consumed milk analysis units (260a, 260b, 260c) during the milk analysis session; and
wherein the transmitted information concerns the estimated remaining number of milk analysis units (260a, 260b, 260c).

3. The control unit (240) according to any one of claims 1-2, configured to:
compare the number of remaining milk analysis units (260a, 260b, 260c) with a second trigger level, which is smaller than the first trigger level; and
output an alert, encouraging an agricultural manager to replace the cassette (130) when the remaining amount of milk analysis units (260a, 260b, 260c) is lower than or equal to the second trigger level.

4. The control unit (240) according to any one of claims 1-3, wherein the milk analysis units (260a, 260b, 260c) are arranged on a tape (250) of the cassette (130).

5. The control unit (240) according to claim 4, configured to:
obtain information concerning a dysfunctional milk analysis unit (260a, 260b, 260c) of the cassette (130) from the memory device (230) of the cassette (130) before performing the test session;
detect that the subsequent milk analysis units (260a, 260b, 260c) on the tape (250) is dysfunctional; and
forward the tape (250) in position for milk analysis action on the next non- dysfunctional milk analysis units (260a, 260b, 260c) on the tape (250).

6. The control unit (240) according to any one of claims 1-5, configured to:
detect when the cassette (130) is used for the first time;
determine a moment in time when the first-time usage of the cassette (130) is detected; and
transmit the determined moment in time to the memory device (230) of the cassette (130), via the first wireless communication device (210), for storage in the memory device (230) of the cassette (130).

7. The control unit (240) according to claim 6, configured to:
determine a current moment in time;
check whether the time difference between the stored moment in time of the memory device (230) of the cassette (130), associated with the first-time usage of the cassette (130), and the current moment in time exceeds a predetermined time limit;
discontinue usage of the cassette (130) when the predetermined time limit is exceeded; and
transmit a blocking sign prohibiting further usage of the cassette (130), via the first wireless communication device (210), for storage in the memory device (230) of the cassette (130), when the predetermined time limit is exceeded.

8. The control unit (240) according to claim 7, configured to:
output an alert, encouraging an agricultural manager to change the cassette (130) when the predetermined time limit is exceeded by the time period, via an output device.

9. The control unit (240) according to any one of claims 1-8, configured to:
activate the first wireless communication device (210) in association with initiating the test session with the milk analysis units (260a, 260b, 260c) of the cassette (130); and/ or
deactivate the first wireless communication device (210) when the information concerning the remaining number of milk analysis units (260a, 260b, 260c) has been transmitted.

10. A system (10) comprising:
a control unit (240) according to any one of claims 1-9;
a cassette (130) comprising a predetermined number of milk analysis units (260a, 260b, 260c) for one time usage, a memory device (230) and a second wireless communication device (220);
a milk analysis apparatus (120) comprising a first wireless communication device (210), for communication with the memory device (230) of the cassette (130) via the second wireless communication device (220) comprised in the cassette (130), and the control unit (240); and
a milk extracting arrangement (110), operating in conjunction with the milk analysis apparatus (120),
wherein the cassette (130) is insertable into the milk analysis apparatus (120), for information exchange with the control unit (240) of the milk analysis apparatus (120) via the first wireless communication device (210) of the milk analysis apparatus (120), when the cassette (130) is inserted into the milk analysis apparatus (120); wherein the memory device (230) is configured to:
receive information concerning remaining number of milk analysis units (260a, 260b, 260c) from the control unit (240) of the milk analysis apparatus (120), via the second wireless communication device (220); and
store the received information.

11. The system (10) according to claim 10, wherein the memory device (230) is configured to:
provide number of remaining milk analysis units (260a, 260b, 260c) of the cassette (130) to the control unit (240), via the second wireless communication device (220).

12. The system (10) according to any one of claim 10 or claim 11, wherein the memory device (230) is configured to:
store information concerning a dysfunctional milk analysis unit (260a, 260b, 260c) of the cassette (130); and
provide the information concerning the dysfunctional milk analysis unit (260a, 260b, 260c) to the control unit (240), via the second wireless communication device (220).

13. The system (10) according to any one of claims 10-12, wherein the memory device (230) is configured to:
receive information concerning a moment in time of a first-time usage of the cassette (130) from the control unit (240) of the milk analysis apparatus (120), via the second wireless communication device (220);
store the received information; and
provide stored information concerning the moment in time of the first-time usage of the cassette (130) to the control unit (240), via the second wireless communication device (220).

14. The system (10) according to any one of claims 10-13, wherein the memory device (230) is configured to:
receive a blocking sign prohibiting further usage of the cassette (130) from the control unit (240) of the milk analysis apparatus (120), via the second wireless communication device (220); and
store the received information.

15. The system (10) according to any one of claims 10-14, wherein the milk analysis units (260a, 260b, 260c) are arranged on a tape (250) of the cassette (130).

## Patentansprüche

1. Steuereinheit (240) für eine Milchanalyseeinrichtung (120), wobei die Milchanalyseeinrichtung (120) eine erste drahtlose Kommunikationsvorrichtung (210) für eine Kommunikation mit einer Speichervorrichtung (230) einer Kassette (130) über eine zweite drahtlose Kommunikationsvorrichtung (220) umfasst, die in der Kassette (130) enthalten ist; wobei die Kassette (130) in die Milchanalyseeinrichtung (120) einführbar ist und eine vorbestimmte Anzahl von Milchanalyseeinheiten (260a, 260b, 260c) für eine einmalige Verwendung umfasst; **dadurch gekennzeichnet, dass** die Steuereinheit (240) konfiguriert ist zum:
Bestimmen einer Anzahl von verbrauchten Milchanalyseeinheiten (260a, 260b, 260c) während einer Milchanalysesitzung;
Übertragen von Informationen, die sich auf die verbrauchte Anzahl von Milchanalyseeinheiten (260a, 260b, 260c) während der Milchanalysesitzung beziehen, an die Speichervorrichtung (230) der Kassette (130), über die erste drahtlose Kommunikationsvorrichtung (210) für eine Speicherung in der Speichervorrichtung (230) der Kassette (130);
Erhalten der Anzahl von verbleibenden Milchanalyseeinheiten (260a, 260b, 260c) der Kassette (130) von der Speichervorrichtung (230) der Kassette (130);
Vergleichen der Anzahl von verbleibenden Milchanalyseeinheiten (260a, 260b, 260c) mit einem ersten Auslösewert; und
Ändern des Testmodus der Milchanalyseeinrichtung (120), wenn weniger Milchanalyseeinheiten (260a, 260b, 260c) als der erste Auslösewert verbleiben.

2. Steuereinheit (240) nach Anspruch 1, wobei eine Berechnung einer verbleibenden Anzahl von Milchanalyseeinheiten (260a, 260b, 260c) umfasst:
Erhalten der Anzahl von verbleibenden Milchanalyseeinheiten (260a, 260b, 260c) der Kassette (130) aus der Speichervorrichtung (230) der Kassette (130) vor dem Durchführen der Testsitzung;
Schätzen der verbleibenden Anzahl von Milchanalyseeinheiten (260a, 260b, 260c) auf der Kassette (130) basierend auf der erhaltenen Anzahl von verbleibenden Milchanalyseeinheiten (260a, 260b, 260c) und der Anzahl von verbrauchten Milchanalyseeinheiten (260a, 260b, 260c) während der Milchanalysesitzung; und
wobei die übertragenen Informationen die geschätzte verbleibende Anzahl von Milchanalyseeinheiten (260a, 260b, 260c) betreffen.

3. Steuereinheit (240) nach einem der Ansprüche 1 bis 2, die konfiguriert ist zum:
Vergleichen der Anzahl von verbleibenden Milchanalyseeinheiten (260a, 260b, 260c) mit einem zweiten Auslösewert, der kleiner als der erste Auslösewert ist; und
Ausgeben einer Warnung, die einen landwirtschaftlichen Manager darin bestärkt, die Kassette (130) zu ersetzen, wenn die verbleibende Menge von Milchanalyseeinheiten (260a, 260b, 260c) geringer als oder gleich dem zweiten Auslösewert ist.

4. Steuereinheit (240) nach einem der Ansprüche 1 bis 3, wobei die Milchanalyseeinheiten (260a, 260b, 260c) auf einem Band (250) der Kassette (130) angeordnet sind.

5. Steuereinheit (240) nach Anspruch 4, die konfiguriert ist zum:
Erhalten von Informationen betreffend eine dysfunktionale Milchanalyseeinheit (260a, 260b, 260c) der Kassette (130) aus der Speichervorrichtung (230) der Kassette (130) vor dem Durchführen der Testsitzung;
Erkennen, dass die nachfolgenden Milchanalyseeinheiten (260a, 260b, 260c) auf dem Band (250) dysfunktional sind; und
Weiterleiten des Bands (250) in Position für eine Milchanalyseaktion an die nächsten nicht dysfunktionalen Milchanalyseeinheiten (260a, 260b, 260c) auf dem Band (250).

6. Steuereinheit (240) nach einem der Ansprüche 1 bis 5, die konfiguriert ist zum:
Erkennen, wenn die Kassette (130) zum ersten Mal verwendet wird;
Bestimmen eines Zeitpunkts, wenn die erstmalige Verwendung der Kassette (130) erkannt wird; und
Übertragen des bestimmten Zeitpunkts an die Speichervorrichtung (230) der Kassette (130) über die erste drahtlose Kommunikationsvorrichtung (210) für die Speicherung in der Speichervorrichtung (230) der Kassette (130).

7. Steuereinheit (240) nach Anspruch 6, die konfiguriert ist zum:
Bestimmen eines aktuellen Zeitpunkts;
Prüfen, ob die Zeitdifferenz zwischen dem gespeicherten Zeitpunkt der Speichervorrichtung (230) der Kassette (130), der der erstmaligen Verwendung der Kassette (130) zugeordnet ist, und dem aktuellen Zeitpunkt eine vorbestimmte Zeitgrenze überschreitet;
Unterbrechen der Verwendung der Kassette (130), wenn die vorbestimmte Zeitgrenze überschritten wird; und
Übertragen eines Blockierzeichens, das eine weitere Verwendung der Kassette (130) verhindert, über die erste drahtlose Kommunikationsvorrichtung (210), für die Speicherung in der Speichervorrichtung (230) der Kassette (130), wenn die vorbestimmte Zeitgrenze überschritten wird.

8. Steuereinheit (240) nach Anspruch 7, die konfiguriert ist zum:
Ausgeben einer Warnung, die einen landwirtschaftlichen Manager darin bestärkt, die Kassette (130) zu wechseln, wenn die vorbestimmte Zeitgrenze durch den Zeitraum überschritten wird, über eine Ausgabevorrichtung.

9. Steuereinheit (240) nach einem der Ansprüche 1 bis 8, die konfiguriert ist zum:
Aktivieren der ersten drahtlosen Kommunikationsvorrichtung (210) in Verbindung mit einem Initiieren der Testsitzung mit den Milchanalyseeinheiten (260a, 260b, 260c) der Kassette (130);
und/oder Deaktivieren der ersten drahtlosen Kommunikationsvorrichtung (210), wenn die Informationen betreffend die verbleibende Anzahl von Milchanalyseeinheiten (260a, 260b, 260c) übertragen wurden.

10. System (10), umfassend:
eine Steuereinheit (240) nach einem der Ansprüche 1 bis 9;
eine Kassette (130), umfassend eine vorbestimmte Anzahl von Milchanalyseeinheiten (260a, 260b, 260c) für eine einmalige Verwendung, eine Speichervorrichtung (230) und eine zweite drahtlose Kommunikationsvorrichtung (220);
eine Milchanalyseeinrichtung (120), umfassend eine erste drahtlose Kommunikationsvorrichtung (210) für die Kommunikation mit der Speichervorrichtung (230) der Kassette (130) über die zweite drahtlose Kommunikationsvorrichtung (220), die in der Kassette (130) enthalten ist, und der Steuereinheit (240); und
eine Milchextraktionsanordnung (110), die zusammen mit der Milchanalyseeinrichtung (120) arbeitet,
wobei die Kassette (130) in die Milchanalysevorrichtung (120) einführbar ist, zum Informationsaustausch mit der Steuereinheit (240) der Milchanalyseeinrichtung (120) über die erste drahtlose Kommunikationsvorrichtung (210) der Milchanalyseeinrichtung (120), wenn die Kassette (130) in die Milchanalyseeinrichtung (120) eingeführt ist; wobei die Speichervorrichtung (230) konfiguriert ist zum:
Empfangen von Informationen bezüglich der verbleibenden Anzahl von Milchanalyseeinheiten (260a, 260b, 260c) von der Steuereinheit (240) der Milchanalyseeinrichtung (120) über die zweite drahtlose Kommunikationsvorrichtung (220); und
Speichern der empfangenen Informationen.

11. System (10) nach Anspruch 10, wobei die zentrale Speichervorrichtung (230) konfiguriert ist zum:
Bereitstellen der Anzahl von verbleibenden Milchanalyseeinheiten (260a, 260b, 260c) der Kassette (130) an die Steuereinheit (240) über die zweite drahtlose Kommunikationsvorrichtung (220).

12. System (10) nach einem der Ansprüche 10 oder 11, wobei die Speichervorrichtung (230) konfiguriert ist zum:
Speichern von Informationen betreffend eine dysfunktionale Milchanalyseeinheit (260a, 260b, 260c) der Kassette (130); und
Bereitstellen der Informationen betreffend die dysfunktionale Milchanalyseeinheit (260a, 260b, 260c) an die Steuereinheit (240) über die zweite drahtlose Kommunikationsvorrichtung (220).

13. System (10) nach einem der Ansprüche 10 bis 12, wobei die Speichervorrichtung (230) konfiguriert ist zum:
Empfangen von Informationen betreffend einen Zeitpunkt einer erstmaligen Verwendung der Kassette (130) von der Steuereinheit (240) der Milchanalyseeinrichtung (120) über die zweite drahtlose Kommunikationsvorrichtung (220);
Speichern der empfangenen Informationen; und
Bereitstellen gespeicherter Informationen betreffend den Zeitpunkt der erstmaligen Verwendung der Kassette (130) an die Steuereinheit (240), über die zweite drahtlose Kommunikationsvorrichtung (220).

14. System (10) nach einem der Ansprüche 10 bis 13, wobei die Speichervorrichtung (230) konfiguriert ist zum:
Empfangen Blockierzeichens, das eine weitere Verwendung der Kassette (130) verhindert, von der Steuereinheit (240) der Milchanalyseeinrichtung (120) über die zweite drahtlose Kommunikationsvorrichtung (220); und
Speichern der empfangenen Informationen.

15. System (10) nach einem der Ansprüche 10 bis 14, wobei die Milchanalyseeinheiten (260a, 260b, 260c) auf einem Band (250) der Kassette (130) angeordnet sind.

## Revendications

1. Unité de commande (240) pour un appareil d'analyse de lait (120), dans laquelle l'appareil d'analyse de lait (120) comprend un premier dispositif de communication sans fil (210), pour une communication avec un dispositif de mémoire (230) d'une cassette (130) par l'intermédiaire d'un second dispositif de communication sans fil (220) compris dans la cassette (130) ; dans laquelle la cassette (130) peut être insérée dans l'appareil d'analyse de lait (120) et comprend un nombre prédéterminé d'unités d'analyse de lait (260a, 260b, 260c) pour une utilisation ponctuelle ; **caractérisée en ce que** l'unité de commande (240) est conçue pour :
déterminer un nombre d'unités d'analyse de lait consommées (260a, 260b, 260c) pendant une session d'analyse de lait ;
transmettre des informations se rapportant au nombre d'unités d'analyse de lait consommées (260a, 260b, 260c) pendant la session d'analyse de lait, au dispositif de mémoire (230) de la cassette (130), par l'intermédiaire du premier dispositif de communication sans fil (210), pour un stockage dans le dispositif de mémoire (230) de la cassette (130) ;
obtenir un nombre restant d'unités d'analyse de lait (260a, 260b, 260c) de la cassette (130) à partir du dispositif de mémoire (230) de la cassette (130) ;
comparer le nombre restant d'unités d'analyse de lait (260a, 260b, 260c) avec un premier niveau de déclenchement ; et
changer le mode de test de l'appareil d'analyse de lait (120) lorsqu'il reste moins d'unités d'analyse de lait (260a, 260b, 260c) que le premier niveau de déclenchement.

2. Unité de commande (240) selon la revendication 1, dans laquelle un calcul d'un nombre restant d'unités d'analyse de lait (260a, 260b, 260c) comprend :
l'obtention d'un nombre restant d'unités d'analyse de lait (260a, 260b, 260c) de la cassette (130) à partir du dispositif de mémoire (230) de la cassette (130) avant de mettre en oeuvre la session de test ;
l'estimation d'un nombre restant d'unités d'analyse de lait (260a, 260b, 260c) sur la cassette (130), sur la base du nombre restant obtenu d'unités d'analyse de lait (260a, 260b, 260c) et du nombre d'unités d'analyse de lait consommées (260a, 260b, 260c) pendant la session d'analyse de lait ; et
dans laquelle les informations transmises concernent le nombre estimé d'unités d'analyse de lait restantes (260a, 260b, 260c).

3. Unité de commande (240) selon l'une quelconque des revendications 1 à 2, conçue pour :
comparer le nombre restant d'unités d'analyse de lait (260a, 260b, 260c) avec un second niveau de déclenchement, qui est plus petit que le premier niveau de déclenchement ; et
délivrer une alerte, encourageant un gestionnaire agricole à remplacer la cassette (130) lorsque la quantité restante d'unités d'analyse de lait (260a, 260b, 260c) est inférieure ou égale au second niveau de déclenchement.

4. Unité de commande (240) selon l'une quelconque des revendications 1 à 3, dans laquelle les unités d'analyse de lait (260a, 260b, 260c) sont agencées sur une bande (250) de la cassette (130).

5. Unité de commande (240) selon la revendication 4, conçue pour :
obtenir des informations concernant une unité d'analyse de lait (260a, 260b, 260c) dysfonctionnelle de la cassette (130) à partir du dispositif de mémoire (230) de la cassette (130) avant de mettre en oeuvre la session de test ;
détecter que les unités d'analyse de lait (260a, 260b, 260c) suivantes sur la bande (250) sont dysfonctionnelles ; et
acheminer la bande (250) en position pour une action d'analyse de lait sur les unités d'analyse de lait (260a, 260b, 260c) non dysfonctionnelles suivantes sur la bande (250).

6. Unité de commande (240) selon l'une quelconque des revendications 1 à 5, conçue pour :
détecter lorsque la cassette (130) est utilisée pour la première fois ;
déterminer une fois où l'utilisation première de la cassette (130) est détectée ; et
transmettre le moment déterminé au dispositif de mémoire (230) de la cassette (130), par l'intermédiaire du premier dispositif de communication sans fil (210), pour un stockage dans le dispositif de mémoire (230) de la cassette (130).

7. Unité de commande (240) selon la revendication 6, conçue pour :
déterminer un moment actuel dans le temps ;
vérifier si la différence de temps entre le moment stocké du dispositif de mémoire (230) de la cassette (130), associée à la première utilisation de la cassette (130), et le moment actuel dans le temps dépasse une limite de temps prédéterminée ;
cesser l'utilisation de la cassette (130) lorsque la limite de temps prédéterminée est dépassée ; et
transmettre un signe de blocage interdisant une utilisation supplémentaire de la cassette (130), par l'intermédiaire du premier dispositif de communication sans fil (210), pour un stockage dans le dispositif de mémoire (230) de la cassette (130), lorsque la limite de temps prédéterminée est dépassée.

8. Unité de commande (240) selon la revendication 7, conçue pour :
délivrer une alerte, encourageant un gestionnaire agricole à changer la cassette (130) lorsque la limite de temps prédéterminée est dépassée par la période de temps, par l'intermédiaire d'un dispositif de sortie.

9. Unité de commande (240) selon l'une quelconque des revendications 1 à 8, conçue pour :
activer le premier dispositif de communication sans fil (210) en association avec le lancement de la session de test avec les unités d'analyse de lait (260a, 260b, 260c) de la cassette (130) ; et/ou désactiver le premier dispositif de communication sans fil (210) lorsque les informations concernant le nombre restant d'unités d'analyse de lait (260a, 260b, 260c) ont été transmises.

10. Système (10) comprenant :
une unité de commande (240) selon l'une quelconque des revendications 1 à 9 ;
une cassette (130) comprenant un nombre prédéterminé d'unités d'analyse de lait (260a, 260b, 260c) pour une utilisation temporaire, un dispositif de mémoire (230) et un second dispositif de communication sans fil (220) ;
un appareil d'analyse de lait (120) comprenant un premier dispositif de communication sans fil (210), pour une communication avec le dispositif de mémoire (230) de la cassette (130) par l'intermédiaire du second dispositif de communication sans fil (220) compris dans la cassette (130), et l'unité de commande (240) ; et
Un agencement d'extraction de lait (110), fonctionnant conjointement avec l'appareil d'analyse de lait (120),
dans lequel la cassette (130) peut être insérée dans l'appareil d'analyse de lait (120), pour un échange d'informations avec l'unité de commande (240) de l'appareil d'analyse de lait (120) par l'intermédiaire du premier dispositif de communication sans fil (210) de l'appareil d'analyse de lait (120), lorsque la cassette (130) est insérée dans l'appareil d'analyse de lait (120) ; dans lequel le dispositif de mémoire (230) est configuré pour :
recevoir des informations concernant un nombre restant d'unités d'analyse de lait (260a, 260b, 260c) provenant de l'unité de commande (240) de l'appareil d'analyse de lait (120), par l'intermédiaire du second dispositif de communication sans fil (220) ; et
stocker les informations reçues.

11. Système (10) selon la revendication 10, dans lequel le dispositif de mémoire (230) est configuré pour :
fournir un nombre restant d'unités d'analyse de lait (260a, 260b, 260c) de la cassette (130) à l'unité de commande (240), par l'intermédiaire du second dispositif de communication sans fil (220).

12. Système (10) selon l'une quelconque de la revendication 10 ou la revendication 11, dans lequel le dispositif de mémoire (230) est configuré pour :
stocker des informations concernant une unité d'analyse de lait (260a, 260b, 260c) dysfonctionnelle de la cassette (130) ; et
fournir les informations concernant l'unité d'analyse de lait (260a, 260b, 260c) dysfonctionnelle à l'unité de commande (240), par l'intermédiaire du second dispositif de communication sans fil (220).

13. Système (10) selon l'une quelconque des revendications 10 ou 12, dans lequel le dispositif de mémoire (230) est configuré pour :
recevoir des informations concernant un moment d'une première utilisation de la cassette (130) provenant l'unité de commande (240) de l'appareil d'analyse de lait (120), par l'intermédiaire du second dispositif de communication sans fil (220) ;
stocker les informations reçues ; et
fournir des informations stockées concernant le moment de la première utilisation de la cassette (130) à l'unité de commande (240), par l'intermédiaire du second dispositif de communication sans fil (220).

14. Système (10) selon l'une quelconque des revendications 10 à 13, dans lequel le dispositif de mémoire (230) est configuré pour :
recevoir un signe de blocage interdisant une utilisation supplémentaire de la cassette (130) de l'unité de commande (240) de l'appareil d'analyse de lait (120), par l'intermédiaire du second dispositif de communication sans fil (220) ; et
stocker les informations reçues.

15. Système (10) selon l'une quelconque des revendications 10 à 14, dans lequel les unités d'analyse de lait (260a, 260b, 260c) sont agencées sur une bande (250) de la cassette (130).
